# EUROPEAN PATENT SPECIFICATION

(11) **EP 0 360 481 B1**
(45) Date of publication and mention of the grant of the patent: **02.03.1994**
(21) Application number: 89309237.9
(22) Date of filing: 12.09.1989
(51) Int. Cl.: C07K 7/06, A61K 37/02

(54) **New pentapeptide and a process for the preparation thereof**
Pentapeptide und Verfahren zu deren Herstellung
Pentapeptides et leur procédé de préparation

(30) Priority: 13.09.1988 NO 884054
(43) Date of publication of application: 28.03.1990
(73) Proprietor: Hafslund Nycomed AS, N-0301 Oslo (NO)
(72) Inventor: Paulsen, Jan Erik,, N-1343 Eiksmarka (NO); Reichelt, Karl-Ludvig,, N-1263 Oslo 12 (NO)
(74) Representative: Matthews, Derek Peter

(56) References cited:
- WO-A-87/00180
- WO-A-88/03535

## Description

The present invention relates to a new pentapeptide which exhibits an inhibitory effect on DNA synthesis and on the mitotic rate in regenerating liver as well as on DNA synthesis and growth of a malignant hepatoma cell-line in vitro and in vivo.

Thus, the pentapeptide may be used in the treatment of hepatic diseases in mammals, particularly humans, having undesired cell growth, as in hepatic cancer. The peptide also affects normal liver cells and may accordingly also be used in other situations in which it is desirable to control the growth of the liver cells.

There are previously known pentapeptides with a cell growth inhibitory activity, as described in WO 87/00180 (PCT/NO86/00041). The pentapeptides described therein inhibit the cell proliferation in squamous epithelia, and are therefore suitable for treatment of undesired cell growth in the epidermis. However, this activity is selective, and the previously known pentapeptides are therefore not equally active with respect to inhibiting undesired cell growth in other organs.

In an article by Jan Erik Paulsen, Karl-L. Reichelt and Anne K. Petersen in Virchows Arch B (1987) 54:152-154, with the title "Purification and characterization of a growth inhibitory hepatic peptide, A preliminary note" it is described that a pentapeptide isolated from mouse liver seems to inhibit DNA synthesis and the mitotic rate in regenerating mouse liver. It was found that after partial hepatectomy the DNA synthesis and the mitotic rate were strongly reduced after treatment with a peptide isolated from mouse liver compared with the controls.

Except from the fact that there is probably a question of a pentapeptide with N-terminal pyroglutamate, nothing is mentioned with respect to which amino acids are included and the sequence thereof. After extensive testing we have found several new pentapeptides with a fully identified structure.

According to the invention there are now provided new pentapeptides of the general formula 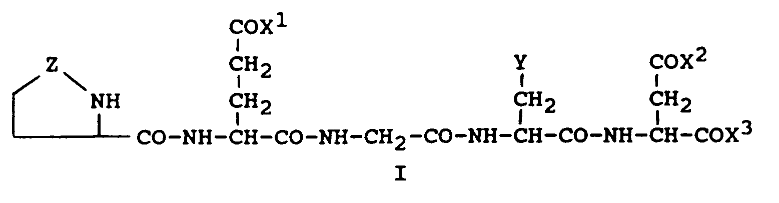
wherein
Z is CH₂ or CO,
Y is H or OH, and
X¹, X² and X³ are each independantly OH or NH₂, provided that X² and X³ are not both NH₂.

The compounds may be prepared in any manner that is suitable for the preparation of peptides. At a late stage of its preparation, the peptide will normally exist as a protected derivative, and the last step or the last steps of the process will be removal of the protective groups (from amino, amido, hydroxyl and/or carboxyl). A particularly suitable process is the socalled solid phase method which is considered suitable for the preparation of oligopeptides and their analogues in a rapid manner and with good yields. In this method the growing peptide chain is kept attached to a solid polymer support, and the synthesis starts by binding the C-terminal amino acid to the polymer. The most common amino acids attached to a polymer support are today commercially available. The next amino acid is then coupled to this polymer-bound amino acid by a repeated cycle with deprotection, washing and coupling. In this manner the entire peptide is built up in a polymer-bound form, and when the entire building up has been finished, the final product is split off from the polymer by means of a suitable reagent. Simultaneously or afterwards remaining protective groups are also removed.

In liquid phase peptide synthesis, the peptide chain starts with the C-terminal amino acid. All side chains except the α-amino group are usually protected with suitable protecting groups stable under the conditions used during the entire synthesis. The α-amino group is either free to react or semiprotected by an easily removable organic or inorganic salt.

To a solution of this C-terminal amino acid (or peptide) the next amino acid to be coupled to the growing peptide chain is added with stirring. All reactive side chains of said next amino acid except the α-carboxy group are usually protected by suitable protecting groups stable under the conditions used during the entire synthesis. The α-carboxy group is either preactivated in any suitable way or ready for in situ activation by any suitable method for coupling to the free amino group of the C-terminal amino acid (or peptide). If the C-terminal amino acid (or peptide) is semiprotected by an organic or inorganic salt, one equivalent of a suitable base is added to provide the free α-amino function.

For in situ activation, any suitable activation method can be used, such as DCC, EEDQ, mixed anhydride, BOP, azide etc.

The preactivated reagents may be symmetrical anhydrides, active esters etc.

Both preactivation and in situ activated carboxylic acid couplings may be assisted by certain chemical compounds added to speed the reaction or to supress/prevent racemization. Such additives may be such compounds as hydroxybenzotriazole, N-hydroxy-succinimide etc.

The resulting peptide is isolated from the reaction mixture by any suitable procedure such as liquid liquid extraction or precipitation. The crude product is usually used without further purification, and purity/identity tests are usually done by TLC. The N-terminal α-amino group of the new peptide is deprotected by a reagent which is able to remove selectively and quantitatively the temporary N-protecting group, leaving the other (side chain) protecting groups intact.

This protected peptide with a free α-amino group is now the C-terminal part of the growing peptide chain, and the entire procedure is repeated with addition of the next amino acid to be added to the N-terminal of the growing peptide chain.

This procedure is repeated until a protected derivative of the entire peptide is obtained.

The free peptide is then prepared by treating the protected derivative of the peptide with reagents able to split off all permanent protecting groups. Examples of such reactions/reagents are:
TFA for medium acid stable protecting groups
Hydrogenolysis over palladium/carbon for benzylic protecting groups
Liquid HF for very acid stable protecting groups.

Thus, the amino acid units that may be part of the pentapeptide, counted from the C-terminal end, are the following:

| | | | |
|---|---|---|---|
| 1) | Aspartic acid | (Asp) | X² = X³ = OH |
| | β-Asparagine | (β-Asn) | X² = NH₂, X³ = OH |
| | α-Asparagine | (α-Asn) | X² = OH, X³ = NH₂ |
| 2) | Alanine | (Ala) | Y = H |
| | Serine | (Ser) | Y = OH |
| 3) | Glycine | (Gly) | |
| 4) | Glutamic acid | (Glu) | X¹ = OH |
| | Glutamine | (Gln) | X¹ = NH₂ |
| 5) | Pyroglutamic acid | (pGlu) | Z = CO |
| | Proline | (Pro) | Z = CH₂ |

Generally, all the amino acids are used in their L-form, except alanine which may also suitably be used in its D-form.

Two of the compounds of formula I have been subjected to further tests in vitro and in vivo. These compounds are:
A: pGlu-Gln-Gly-Ser-β-Asn
   (Z = CO, Y = OH, X¹ = NH₂, X² = NH₂, X³ = OH)
B: pGlu-Glu-Gly-Ser-β-Asn
   (Z = CO, Y = OH, X¹ = OH, X² = NH₂, X³ = OH) (all amino acids in L-configuration).

The following table illustrates how compound A inhibited the regenerative increase of liver weight in mice after 70% hepatectomy. Compound A was injected daily i.p. at 12oo a.m. in saline. Controls were treated with saline only.

| Day after 70% hepatectomy | pmole of peptide per animal | Liver wet weight % of control | p |
|---|---|---|---|
| 4 | 0 | 100 | |
| 4 | 1 | 101 ± 7 | |
| 4 | 10 | 79 ± 6 | 0.005 |
| 4 | 100 | 77 ± 5 | 0.0025 |
| N = 8 ± SD | | | |

It was further found that this peptide also inhibits DNA synthesis and proliferation of MH₁C₁, a clonal strain from a Morris transplantable hepatoma in vitro.

Compound B was given to mice subjected to 70% hepatectomy, and the mitotic rate was measured 5 hours after the administration. It was then found that the mitotic rate was about 40% of the mitotic rate of the controls as it will appear from the following table:

| Peptide pmole per animal | Mitotic rate % of controls | p |
|---|---|---|
| 0 (control) | 100 | |
| 1 | 52 ± 9 | < 0.0025 |
| 10 | 39 ± 11 | < 0.0005 |
| 100 | 37 ± 9 | < 0.0005 |
| N = 6 ± SD | | |

Four hours after treatment with the same peptide B, it was also found that the mitotic rate was reduced to about 50% of the rate in the controls 48 hours after CCl₄ intoxication.

| Peptide pmole per animal | Mitotic rate % of controls | p |
|---|---|---|
| 0 (control) | 100 | |
| 1 | 90 ± 13 | |
| 10 | 49 ± 10 | < 0.025 |
| 100 | 74 ± 12 | |
| N = 3 ± SD | | |

Peptide B reduces the total number of rat hepatoma cells in vitro to about 70% of the controls 72 hours after the peptide addition. This will appear from the following table:

| Peptide concentration log M | Total number of cells % of controls | p |
|---|---|---|
| 0 (control) | 100 | |
| -14 | 79 ± 6 | < 0.005 |
| -11 | 71 ± 5 | < 0.0025 |
| - 8 | 80 ± 7 | < 0.025 |
| N = 6 ± SD | | |

To test the activity of compound B on the malignant hepatoma cell line also in vivo four groups of each 8 animals were used, and a predetermined number of malign cells were injected i.v., and the animals were then treated by using three different concentrations of compound B.

In this experiment the metastasis spreading to the lungs was measured, and a great reduction could be observed in the treated groups.

### Experimental

Immediately after the injection of peptide B, Buffalo female rats, 30 days old, were injected with 10⁴ rat hepatoma cells (MH₁C₁) in a tail vein. The rats then received the peptide i.p. three times per week during the experiment (a total of 12 injections). Four weeks after the experiment started, the lungs containing metastases from the hepatoma cell injection, were weighed (wet weight).

### Results

Peptide B reduces the weight of the lung (lung + metastases) to about 40% of the controls. The reduction of the tumor mass is even greater when the weight of the normal lung is subtracted. The tests are illustrated in the following table:

| Peptide B pmole per animal | Weight of lung + metastases % of controls | p |
|---|---|---|
| 0 (cntrol) | 100 | |
| 2 | 55 ± 37 | < 0.01 |
| 20 | 54 ± 24 | < 0.005 |
| 200 | 42 ± 8 | < 0.0005 |
| N = 8 ± SD | | |

When the weight of the normal lung tissue is taken into consideration and is subtracted from the weights found, the weight of the tumor is found as it will appear from the following table, calculated as % of control:

| Peptide B, pmole per animal | Tumor weight, % of control |
|---|---|
| 2 | 45 |
| 20 | 43 |
| 200 | 29 |

In view of the above described properties, the new pentapeptides of formula I are particularly suitable in the treatment of hepatic cancer, and can be incorporated in common formulations for parenteral administration. Suitable doses will depend on the actual peptide, the patient and the administration route and composition form. However, the doses used in the above examples indicate relevant doses.

The preparation of the peptides of formula I is further illustrated in the following:
The peptides were prepared using the solid phase method according to Merrifield. A known amount of a resin support was placed in a reaction vessel and mixed with 150 ml of the following solvents in the given order. All washings took place for 1 minute if nothing else is stated. All the amino acids used were in the L-configuration. Asn was β-Asn.
1. Methylene chloride - three times
2. 40% trifluoroacetic acid in methylene chloride
3. 40% trifluoroacetic acid in methylene chloride - 30 minutes - once
4. Methylene chloride - once
5. Ethanol - once
6. Methylene chloride - twice
7. 10% triethylamine in methylene chloride - once
8. 10% triethylamine in methylene chloride - 10 minutes - once
9. Methylene chloride - three times
10. Coupling with the amino acid.

Each amino acid was coupled in turn, starting with the carboxyl terminal amino acid. Equal equivalents of the Boc amino acid and the DCC were added in excess based on the resin used. After each coupling the resin was examined by the Kaiser-test to ascertain that the reaction was complete. Deprotection was carried out with 40% TFA, and this was also controlled by the Kaiser-test. The synthesis was considered complete when the last amino acid was successfully coupled.
Starting materials used in the synthesis of pGlu-Gln-Gly-Ser-Asn:
1. para-methyl-benzhydrilamine resin
2. Boc-Asn-α-O-benzyl
3. Boc-Ser (Bzl)
4. Boc-Gly
5. Boc-Gln (Xan)
6. p-Glu

In the synthesis of pGlu-Glu-Gly-Ser-Asn, Boc-Glu(OcHex) is used instead of Boc-Gln(Xan).
The starting materials used in the synthesis of pGlu-Gln-Gly-Ser-Asp:
1. Boc-Asp(OBzl)-resin
2. Boc-Ser (Bzl)
3. Boc-Gly
4. Boc-Gln (Xan)
5. pGlu

For the preparation of pGlu-Glu-Gly-Ser-Asp, Boc-Glu(OcHex) is used in place of Boc-Gln(Xan).

### HF-decomposition:

The peptide attached to the resin was placed in a Kel-F-vessel and cooled to 0°C. Anisole was added as scavenger, and liquid hydrogen fluoride was introduced into the system and mixed with the resin for 45 minutes. Hydrogen fluoride was evaporated under vacuum after a standard procedure prescribed by the supplier. The resin was extracted with ether to remove scavenger and lipophilic byproducts. The crude peptide was extracted into acetic acid and water.

### Purification:

Purification of the above peptides was carried out as follows:
1. An open glass column was packed with C-18 resin washed with acetonitrile.
2. The column was pretreated with buffer A which was 0.1% TFA, using 500-1000 ml.
3. The peptide was dissolved and added to the top of the column.
4. A linear gradient was started from 0% B-buffer to 100% B in 40 minutes, buffer B being 60% acetonitrile in A.
5. The peptide was detected by TLC. It was run on HPLC, and the purest fractions were collected and lyophilized.

### Explanations:

- DCC =: dicyclohexylcarbodiimide
- Bzl =: benzyl
- Xan =: xanthyl
- TFA =: trifluoroacetic acid
- OcHex =: cyclohexyloxy
- Boc =: t-butoxycarbonyl
- EEDQ =: N-ethyloxycarbonyl-2-ethyloxy)-1-dihydroquinoline
- BOP =: Benzotriazol-1-yl-oxy-tris-(dimethylamino)phosphonium-hexafluorophosphate

### Analysis:

### 1) Analysis of pGlu-Gln-Gly-Ser-Asn m.w. 515.44

### Thin layer chromatography:

Silica Fₘ (nBuOH:Pyr:HOAc:H₂O 15:15:3:12) o-tolidine
Results: Main spot with a distinct lower spot and a negligible upper spot. R_{f} = 0.28
Silica 1:1:1:1 (nBuOH:EtOAc:HOAc:H₂O) o-tolidine
Results: Major spot with a negligible upper spot and a negligible lower spot. R_{f} = 0.22

### Electrophoresis:

Whatman 3MM, pH 3.5 (Pyr:Acetone); 1500V. 1 h. o-tolidine
Results: Major spot migrates towards the anode with a negligible lower spot.
R_{f} = 0.25 with picric acid as reference.

| Amino acid analysis: % of peptide 91,1 % | | |
|---|---|---|
| | Theory | Found |
| Asp | 1 | 1.09 |
| Ser | 1 | 0.92 |
| Glu | 2 | 2.01 |
| Gly | 1 | 0.98 |

### 2) Analysis of pGlu-Glu-Gly-Ser-Asn m.w. 516.42

### Thin layer chromatography:

Silica gel Fₘ (nBuOH:Pyr:HOAc:H₂O, 15:15:3:12) o-tolidine
Silica gel 1:1:1:1 (nBuOH:EtOAc:HOAc:H₂O) o-tolidine
Results: Fₘ: Major spot with a negligible lower spot
R_{f} = 0.44
1:1:1:1: Major spot with a negligible lower spot.
R_{f} = 0.39

### Electrophoresis:

Whatman 3MM, pH 1.9 (HCOOH:Acetone); 1000V. 1 h. o-tolidine
Results: Single spot migrating towards the anode
R_{f} = 0.40 with picric acid as reference.

| Amino acid analysis: % of peptide 90.1% | | |
|---|---|---|
| | Theory | Found |
| Asp | 1 | 0.94 |
| Ser | 1 | 0.95 |
| Glu | 2 | 2.08 |
| Gly | 1 | 0.98 |

### 3) Analysis of pGlu-Gln-Gly-Ser-Asp m.w. 516.19

### Thin layer chromatography:

Silica Fₘ (nBuOH:Pyr:HOAc:H₂O, 15;15:3:12) o-tolidine
Results: Major spot with a faint upper spot and a negligible lower spot. R₂ = 0.27
Silicagel 1:1:1:1 (nBuOH:EtOAc:HOAc:H₂O) o-tolidine
Results: Major spot with a negligible upper spot and a negligible lower spot. R₂ = 0.32

| Amino acid analysis: % of peptide 87.4% | | |
|---|---|---|
| | Theory | Found |
| Asp | 1 | 0.98 |
| Ser | 1 | 0.91 |
| Glu | 2 | 2.06 |
| Gly | 1 | 0.97 |

### 4) Analysis of pGlu-Glu-Gly-Ser-Asp m.w. 517.18

### Thin layer chromatography:

Silica Fₘ (nBuOH:Pyr:HOAc:H₂O, 15:15:3:12) o-tolidine
Results: Major spot with a negligible upper spot and a negligible lower spot. R_{f} = 0.22
Silica 1:1:1:1 (nBuOH:EtOAc:HOAc:H₂O) o-tolidine
Results: Major spot with a negligible lower spot.
R_{f} = 0.34

| Amino acid analysis: % of peptide 90.7% | | |
|---|---|---|
| | Theory | Found |
| Asp | 1 | 1.00 |
| Ser | 1 | 0.89 |
| Glu | 2 | 2.04 |
| Gly | 1 | 0.96 |

### pGlu-Glu-Gly-Ser-Asp

The above pentapeptide is prepared in solution by the active ester method with minimum purification of the protected intermediates.

The reactants
Asp(OBzl)₂
Boc-Ser(Bzl)-OSu
Boc-Gly-OSu
Boc-Glu(γ-OBzl)-OSu
Cbz-pGlu-OSu
are all commercially available compounds.

### Boc-Ser(Bzl)-Asp(OBzl)₂: (A)

Boc-Ser(Bzl)-OSu (1.2 eq.) dissolved in a minimum of DMF is added dropwise to a stirred solution of Asp(OBzl)₂ (1 eq.) in a minimum of DMF. The reaction is monitored by TLC/Ninhydrin. Negative Ninhydrin test indicates total consumption of the amine compound.

The solvent is evaporated in vacuo; the residue is dissolved in CH₂Cl₂ and extracted with:

| | |
|---|---|
| 0.05N H₂SO₄ | (2x) |
| 1M NaHCO₃ | (2x) |
| Brine | (1x) |
| H₂O | (1x) |

After drying over MgSO₄, filtering and evaporation of solvent in vacuo, the residue is dissolved in a minimum of CH₂Cl₂ and triturated with ether/pet.ether and stored at +4°C over night. The precipitate is collected and washed with cold ether/pet.ether and dried in vacuo.
Crude yield: 95%.

### Ser(Bzl)-Asp(OBzl)₂*TFA (B)

The crude A is dissolved in icecold CH₂Cl₂ and diluted with an equal volume of TFA. The reaction is monitored by TLC. After 30 min. the solvent is evaporated in vacuo, the residue is resuspended in CH₂Cl₂ and evaporated to dryness in vacuo. The crude product is used without further purification.

### Boc-Gly-Ser(Bzl)-Asp(OBzl)₂ (C)

Boc-Gly-OSu (1.2 eq.) dissolved in a minimum of DMF is added dropwise to a stirred solution of B (1 eq.) and NEM (1 eq.) in a minimum of DMF. The reaction is monitored with TLC/Ninhydrin. After 2h, further 0.2 eq. of Boc-Gly-OSu is added, and stirring is continued over night.

The reaction mixture is now Ninhydrin negative on TLC, and the crude product is worked up with the same procedure as described for A. After trituration with ether/pet.ether, the crude product is used without further purification.
Crude yield: 85%.

### Gly-Ser(Bzl)-Asp(OBzl)₂*TFA (D)

The crude product C is treated with an icecold solution of CH₂Cl₂/TFA 1:1 for 30 min. TLC indicates full consumption of C. The solvent is evaporated in vacuo; the residue is redissolved in MeOH and evaporated to dryness in vacuo. Crude D is used without further purification.

### Boc-Glu(γ-OBzl)-Gly-Ser(Bzl)-Asp(OBzl)₂ (E)

Boc-Glu(γ-OBzl)-OSu (1.3 eq.) dissolved in a minimum of DMF is added to a stirred solution of D (1 eq.) and NEM (1 eq.) in a minimum of DMF. The reaction is monitored by TLC/Ninhydrin.

The solvent is evaporated in vacuo, and the crude product is worked up with the procedure described for A.
Crude yield: 90%.
The crude product is used without further purification.

### Glu(γ-OBzl)Gly-Ser(Bzl)-Asp(OBzl)₂*TFA (F)

The crude product E is treated with an icecold solution of CH₂Cl₂/TFA 1:1 for 30 min. TLC indicates full consumption of E. The solvent is evaporated in vacuo; the residue is redissolved in MeOH and evaporated to dryness in vacuo. The crude F is used without further purification.

### Cbz-pGlu-Glu(γ-OBzl)-Gly-Ser(Bzl)-Asp(OBzl)₂ (G)

Cbz-pGlu-OSu (1.3 eq.) dissolved in a minimum of DMF is added dropwise to a solution of F (1 eq.) and NEM (1 eq.) in a minimum of DMF. The reaction is monitored by TLC/Ninhydrin, and stirring is continued over night.

The solvent is evaporated in vacuo, and the crude product is worked up as described for A. The crude product is purified by flash chromatography with CHCl₃/MeOH as solvent.
Total yield from A: 33%.

### pGlu-Glu-Gly-Ser-Asp (H)

The purified product G is dissolved in MeOH, and 10% Pd/C and ammonium formate (5 eq.) are added. The reaction is monitored by TLC and after 45 min. the starting material is totally consumed and TLC is UV₂₅₄ negative. The catalyst is removed by filtration, the solvent is evaporated in vacuo. Ammonium formate is removed by lyophilization, and the crude product is purified and identified as compound 4.

### Additional explanations:

- DMF =: dimethylformamide
- Cbz =: carbobenzoxy
- Su =: succinimido
- NEM =: N-ethyl morpholine

## Claims (Claims for the following Contracting State(s): AT, BE, CH, DE, FR, GB, IT, LI, LU, NL, SE)

1. New pentapeptide, characterized in that it has the formula: 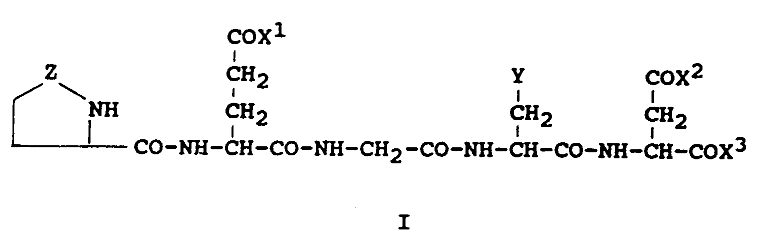 wherein
Z is CH₂ or CO,
Y is H or OH, and
X¹, X² and X³ are each independently OH or NH₂, provided that X² and X³ not both NH₂.

2. Pentapeptide according to claim 1, characterized in that Z is CO, Y is OH, X¹ is OH, X² is NH₂ and X³ is OH.

3. Pentapeptide according to claim 1, characterized in that Z is CO, Y is OH, X¹ is OH, X² is OH and X³ is OH.

4. Composition for inhibiting uncontrolled cell growth in liver, characterized in that it as the active component comprises a compound of the formula 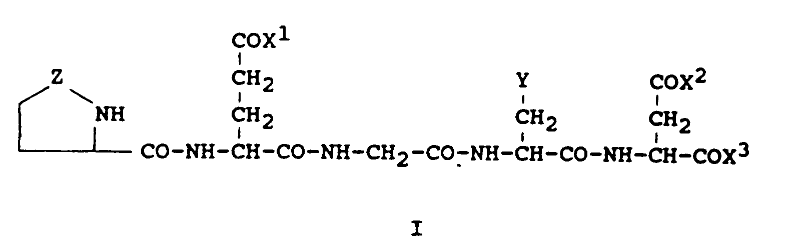 wherein
Z is CH₂ or CO,
Y is H or OH, and
X¹, X² and X³ are each independantly OH or NH₂, provided that X² and X³ are not both NH₂.

5. Composition according to claim 4, characterized in that it comprises a compound of formula I wherein Z is CO, Y is OH, X¹ is OH, X² is NH₂ and X³ is OH.

6. Composition according to claim 4, characterized in that it comprises a compound of formula I wherein Z is CO, Y is OH, X¹ is OH, X² is OH and X³ is OH.

7. Process for the preparation of a pentapeptide of the formula 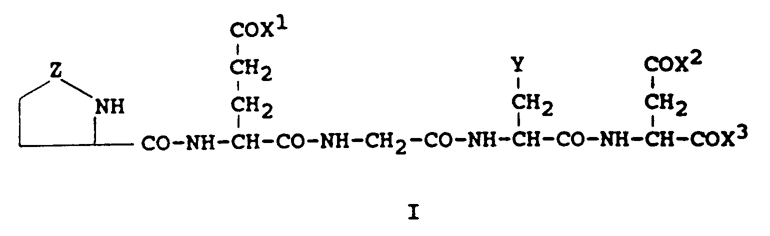 wherein
Z is CH₂ or CO,
Y is H or OH, and
X¹, X² and X³ are each independantly OH or NH₂, provided that X² and X³ are not both NH₂,
characterized in that a protected derivative thereof is deprotected.

8. Process according to claim 7, characterized in that the pentapeptide, optionally in a protected form, is built up by solid phase peptide synthesis wherein the C-terminal amino acid is first attached to a solid support and the following amino acids are then coupled thereto in the desired sequence, and the protective groups are removed.

## Claims (Claims for the following Contracting State(s): ES, GR)

1. Process for the preparation of a pentapeptide of the formula 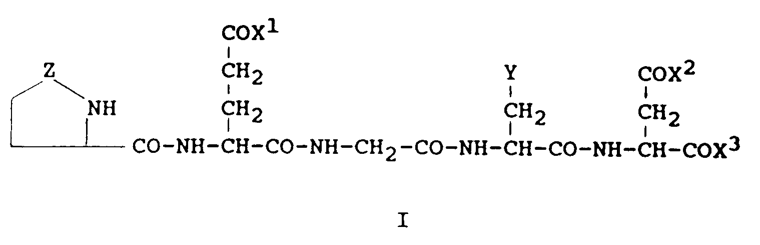 wherein
Z is CH₂ or CO,
Y is H or OH, and
X¹, X² and X³ are each independently OH or NH₂, provided that X² and X³ are not both NH₂,
characterised in that a protected derivative thereof is deprotected.

2. Process according to claim 1, characterised in that the pentapeptide, optionally in a protected form, is built up by solid phase peptide synthesis wherein the C-terminal amino acid is first attached to a solid support and the following amino acids are then coupled thereto in the desired sequence, and the protective groups are removed.

3. Process according to claim 1 or claim 2, characterised in that Z is CO, Y is OH, X¹ is OH, X² is NH₂ and X³ is OH.

4. Process according to claim 1 or claim 2, characterised in that Z is CO, Y is OH, X¹ is OH, X² is OH and X³ is OH.

## Patentansprüche (Patentansprüche für folgende(n) Vertragsstaat(en): AT, BE, CH, DE, FR, GB, IT, LI, LU, NL, SE)

1. Pentapeptide,
**gekennzeichnet** durch
die Formel 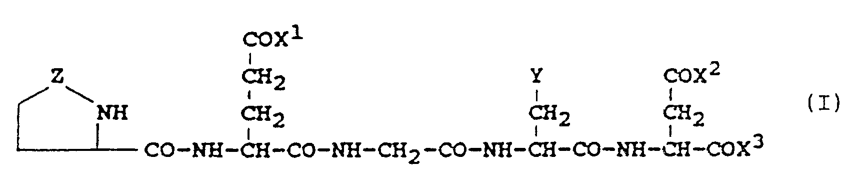 in der Z CH₂ oder CO ist,
Y H oder OH ist und
X¹, X² und X³ unabhängig voneinander OH oder NH₂ sind, und zwar mit der Maßgabe, daß X² und X³ nicht beide NH₂ sind.

2. Pentapeptid nach Anspruch 1,
dadurch **gekennzeichnet,**
daß Z CO ist, Y OH ist, X¹ OH ist, X² NH₂ ist und X³ OH ist.

3. Pentapeptid nach Anspruch 1,
dadurch **gekennzeichnet**,
daß Z CO ist, Y OH ist, X¹ OH ist, X² OH ist, und X³ OH ist.

4. Zusammensetzung zur Unterdrückung von unkontrolliertem Zellwachstum in der Leber,
dadurch **gekennzeichnet**,
daß sie als aktive Komponente eine Substanz der Formel 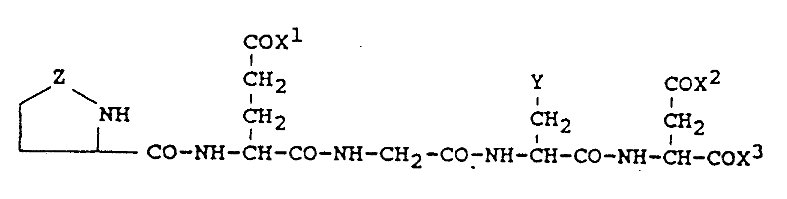 enthält, in der
Z CH₂ oder CO ist,
Y H oder OH ist, und
X¹, X² und X³ jeweils unabhängig voneinander OH oder NH₂ sind, und zwar mit der Maßgabe, daß X² und X³ nicht beide gleichzeitig NH₂ sind.

5. Zusammensetzung nach Anspruch 4,
dadurch **gekennzeichnet**,
daß sie eine Substanz der Formel I enthält, in der Z CO ist, Y OH ist, X¹ OH ist, X² NH₂ ist und X³ OH ist.

6. Zusammensetzung nach Anspruch 4,
dadurch **gekennzeichnet**,
daß sie eine Substanz der Formel I enthält, in der Z CO ist, Y OH ist, X¹ OH ist, X² OH ist und X³ OH ist.

7. Verfahren zur Herstellung eines Pentapeptids der Formel 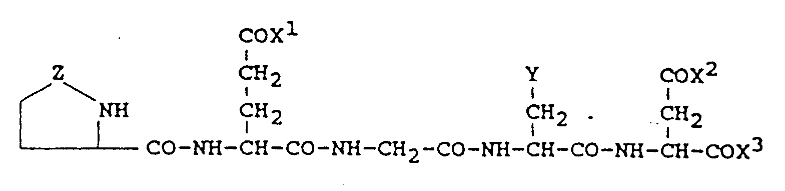 in der Z CH₂ oder CO ist,
Y H oder OH ist, und
X¹, X² und X³ jeweils unabhängig voneinander OH oder NH₂ sind, und zwar mit der Maßgabe, daß X² und X³ nicht beide gleichzeitig NH₂ sind,
dadurch **gekennzeichnet**,
daß von einem geschützten Derivat des Pentapeptids die Schutzgruppen entfernt werden.

8. Verfahren nach Anspruch 7,
dadurch **gekennzeichnet**,
daß das Pentapeptid, wahlweise in seiner geschützten Form, durch Peptidsynthese in fester Phase aufgebaut wird, wobei die Aminosäure der endständigen Kohlenstoffgruppe zunächst an einen festen Träger gebunden wird, und die folgenden Aminosäuren dann in der benötigten Sequenz an diese gebunden werden, und daß dann die Schutzgruppen entfernt werden.

## Patentansprüche (Patentansprüche für folgende(n) Vertragsstaat(en): ES, GR)

1. Verfahren zur Herstellung eines Pentapeptids der Formel 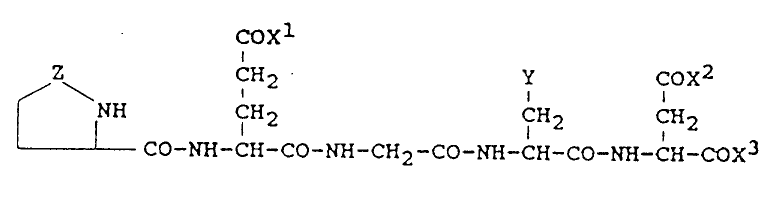 in der Z CH₂ oder CO ist,
Y H oder OH ist und
X¹, X² und X³ jeweils unabhängig voneinander OH oder NH₂ sind, und zwar mit der Maßgabe, daß X² und X³ nicht beide NH₂ sind
dadurch **gekennzeichnet**,
daß von einem geschützten Derivat des Pentapeptids die Schutzgruppen entfernt werden.

2. Verfahren nach Anspruch 1,
dadurch **gekennzeichnet**,
daß das Pentapeptid, wahlweise in seiner geschützten Form, durch Peptidsynthese in fester Phase aufgebaut wird, wobei die Aminosäure der endständigen Kohlenstoffgruppe zunächst an einen festen Träger gebunden wird, und die folgenden Aminosäuren dann in der benötigten Sequenz an diese gebunden werden, und daß dann die Schutzgruppen entfernt werden.

3. Verfahren nach Anspruch 1 oder nach Anspruch 2,
dadurch **gekennzeichnet**,
daß Z CO ista, Y OH ist, X¹ OH ist, X² NH₂ ist und X³ OH ist.

4. Verfahren nach Anspruch 1 oder nach Anspruch 2,
dadurch **gekennzeichnet**,
daß Z CO ist, Y OH ist, X¹ OH ist, X² OH ist, und X³ OH ist.

## Revendications (Revendications pour l'(les) Etat(s) contractant(s) suivant(s): AT, BE, CH, DE, FR, GB, IT, LI, LU, NL, SE)

1. Un nouveau pentapeptide caractérisé en ce qu'il est représenté par la formule: 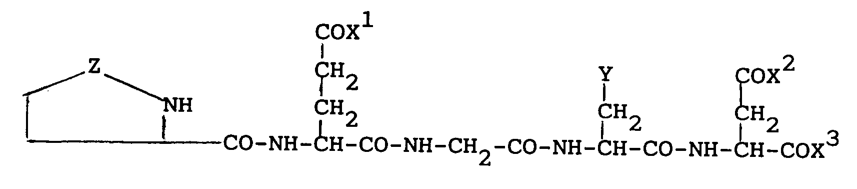 dans laquelle:
Z représente CH₂ ou CO,
Y représente H ou OH, et
X¹, X² et X³ représentent, indépendamment les uns des autres, OH ou NH₂, à condition que X² et X³ ne représentent pas tous les deux NH₂.

2. Le pentapeptide selon la revendication 1, caractérisé en ce que Z représente CO, Y représente OH, X¹ représente OH, X² représente NH₂ et X³ représente OH.

3. Le pentapeptide selon la revendication 1, caractérisé en ce que Z représente CO, Y représente OH, X¹ représente OH, X² représente OH et X³ représente OH.

4. Une composition permettant d'inhiber la croissance non contrôlée des cellules hépatiques, caractérisée en ce que la composition comprend en tant que principe actif un composé représenté par la formule: 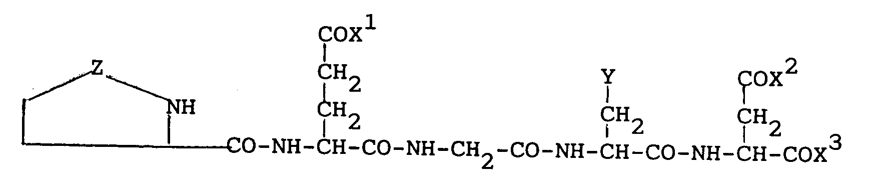 dans laquelle:
Z représente CH₂ ou CO,
Y représente H ou OH, et
X¹, X² et X³ représentent, indépendamment les uns des autres, OH ou NH₂, à condition que X² et X³ ne représentent pas tous les deux NH₂.

5. La composition selon la revendication 4, caractérisée en ce qu'elle comprend un composé représenté par la formule I, dans laquelle Z représente CO, Y représente OH, X¹ représente OH, X² représente NH₂ et X³ représente OH.

6. La composition selon la revendication 4, caractérisée en ce qu'elle comprend un composé de formule I, dans laquelle Z représente CO, Y représente OH, X¹ représente OH, X² représente OH et X³ représente OH.

7. Un procédé de préparation d'un pentapeptide représenté par la formule: 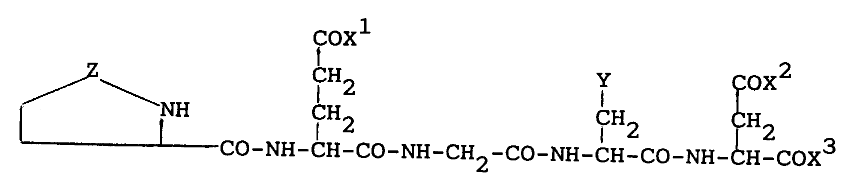 dans laquelle:
Z représente CH₂ ou CO,
Y représente H ou OH, et
X¹, X² et X³ représentent, indépendamment les uns des autres, OH ou NH₂, à condition que X² et X³ ne représentent pas tous les deux NH₂,
caractérisé en ce que le dérivé protégé de ce composé est déprotégé.

8. Le procédé selon la revendication 7, caractérisé en ce que le pentapeptide, éventuellement sous une forme protégée, est synthétisé par une synthèse peptidique en phase solide, dans laquelle l'acide aminé C-terminal est dans un premier temps lié à un support solide, puis les acides aminés suivants sont couplés à ce dernier selon la séquence qui est désirée et les groupes protecteurs sont éliminés.

## Revendications (Revendications pour l'(les) Etat(s) contractant(s) suivant(s): ES, GR)

1. Un procédé de préparation d'un pentapeptide représenté par la formule: 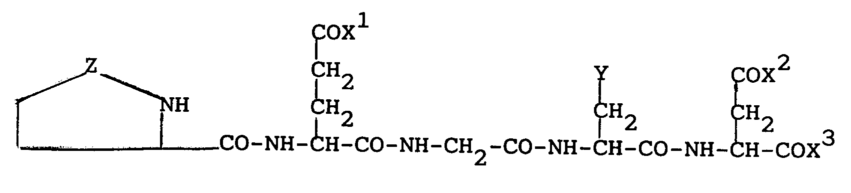 dans laquelle:
Z représente CH₂ ou CO,
Y représente H ou OH, et
X¹, X² et X³ représentent, indépendamment les uns des autres, OH ou NH₂, à condition que X² et X³ ne représentent pas tous les deux NH₂,
caractérisé en ce que le dérivé protégé de ce composé est déprotégé.

2. Le procédé selon la revendication 1, caractérisé en ce que le pentapeptide, éventuellement sous une forme protégée, est synthétisé par une synthèse peptidique en phase solide, dans laquelle l'acide aminé C-terminal est dans un premier temps lié à un support solide, puis les acides aminés suivants sont couplés à ce dernier selon la séquence qui est désirée et les groupes protecteurs sont éliminés.

3. Le procédé selon la revendication 1 ou 2, caractérisé en ce que Z représente CO, Y représente OH, X¹ représente OH, X² représente NH₂ et X³ représente OH.

4. Le procédé selon la revendication 1 ou 2, caractérisé en ce que Z représente CO, Y représente OH, X¹ représente OH, X² représente OH et X³ représente OH.
